# EUROPEAN PATENT APPLICATION

(11) **EP 1 092 773 A2**
(43) Date of publication of application: **18.04.2001**
(21) Application number: 00308948.9
(22) Date of filing: 11.10.2000
(51) Int. Cl.: C12N 15/25, C07K 14/545, C12N 15/63, C12N 5/10, C07K 16/24, A61K 38/20, A61K 39/395

(54) **Polypeptide and uses thereof**

(30) Priority: 15.10.1999 JP 29449399
(71) Applicant: KABUSHIKI KAISHA HAYASHIBARA SEIBUTSU KAGAKU KENKYUJO, Okayama-shi Okayama (JP)
(72) Inventor: Ushio, Shimpei, Okayama-shi, Okayama (JP); Nukada, Yoshiyuki, Okayama-shi, Okayama (JP); Yamamoto, Kozo, Okayama-shi, Okayama (JP); Kurimoto, Masashi, Okayama-shi, Okayama (JP)
(74) Representative: Daniels, Jeffrey Nicholas

(57) **Abstract**

Disclosed are a polypeptide comprising the amino acid sequence of SEQ ID NO:5 or an amino acid sequence which has a homology of at least 75% with respect to the amino acid sequence of SEQ ID NO:5 and contains the amino acid sequence of SEQ ID NO:7 as an internal partial amino acid sequence; a DNA comprising either a nucleotide sequence which has the nucleotide sequence of SEQ ID NO:4, a nucleotide sequence which has a homology of at least 75% with respect to the nucleotide sequence of SEQ ID NO:4 and contains the nucleotide sequence of SEQ ID NO:8 at the 5'-terminus, or a complementary nucleotide sequence thereunto; an antibody which immunologically reacts with the polypeptide; and a method for detecting the polypeptide.

## Description

The present invention relates to a novel polypeptide, DNA encoding the polypeptide, and preparation and uses thereof.

Interleukin (hereinafter called "IL-1"), a cytokine produced by nucleated cells, is an important polypeptide for living bodies because it relates to almost most of the organs and to the maintenance of the system of homeostasis. Examples of the biological activities of IL-1 are as follows: Activation of natural killer cells, activation of neutrophils, alteration of the property of blood vessel endothelial cell surface, acceleration of the disintegration of bone and cartilage, hemopoietic acceleration, maturation induction of precursor B-cells, proliferation induction of matured B-cells, activation of T-cells, induction of IL-2 production, induction of cytokine production, cytotoxic/cytostatic activity against some tumor cells, acceleration of the cell division of fibroblasts, and proliferation-accelerating action of tumor cells in soft agar. Insofar two types of IL-1, i.e., IL-1α and IL-1β, were reported. It was revealed that these IL-1α and IL-1β bind to the same cell surface receptor and have substantially the same biological activities, however, they have only a homology of about 45% with respect to their nucleotide sequences and have a homology of about 26% with respect to their amino acid sequences. There has been found the existence of IL-1 receptor antagonist (IL-1Ra) and IL-1 receptor β (IL-1Raβ) as IL-1 related substances. Under these circumstances, the potential existence of an IL-1 related substance other than IL-1α, IL-1β, IL-1Ra and IL-1Raβ, i.e., IL-1 family, has been said, but there is no such a report. Since such an IL-1 related substance is useful for elucidating the immune system of living bodies and exploring novel pharmaceuticals, the screening and establishment of the substance has been greatly expected.

### Summary of the Invention

The first aim of the present invention is to provide a novel polypeptide (hereinafter called "the present polypeptide"), as an IL-1 related substance, which differs from conventional IL-1α, IL-1β, IL-1Ra and IL-1Raβ.

The second aim of the present invention is to provide a receptor protein which binds to the present polypeptide.

The third aim of the present invention is to provide a DNA which encodes the present polypeptide.

The fourth aim of the present invention is to provide an autonomously-replicable recombinant DNA which comprises the above DNA and an autonomously-replicable vector.

The fifth aim of the present invention is to provide a transformant obtainable by introducing the above recombinant DNA into an appropriate host.

The sixth aim of the present invention is to provide a process for producing the present polypeptide.

The seventh aim of the present invention is to provide an antibody which immunologically reacts with the present polypeptide.

The eighth aim of the present invention is to provide a pharmaceutical composition which comprises the present polypeptide as an effective ingredient.

The ninth aim of the present invention is to provide a pharmaceutical composition which comprises an antibody that immunologically reacts with the present polypeptide.

To achieve the above aims, the present inventors widely screened mRNAs derived from established animal cells using cDNAs of IL-1α, IL-1β and IL-1Ra. As a result, they succeeded to isolate the present polypeptide, which comprises the amino acid sequence of SEQ ID NO:5, from HPB-MLT cells, FERM BP-2430, an established human T-cell line. As mentioned above, the present polypeptide was obtained based on the cDNAs of IL-1α, IL-1β and IL-lRa, however, the homology of amino acid sequences between the present polypeptide and conventional IL-1α, IL-1β or IL-1Ra is merely less than 20%, revealing that the present polypeptide is a novel polypeptide which belongs to IL-1 family but differs from conventionally known IL-1 family. The present invention was made based on this finding.

Accordingly, the present invention provides a novel polypeptide derived from HPB-MLT cells, FERM BP-2430, an established human T-cell line.

The present invention further provides a receptor protein which binds to the present polypeptide.

The present invention further provides a DNA which encodes the present polypeptide.

The present invention further provides an autonomously-replicable recombinant DNA which comprises the above DNA and an autonomously-replicable vector.

The present invention further provides a transformant obtainable by introducing the above recombinant DNA into an appropriate host.

The present invention further provides a process for producing the present polypeptide.

The present invention further provides an antibody which immunologically reacts with the present polypeptide.

The present invention further provides a pharmaceutical composition which comprises the present polypeptide as an effective ingredient.

The present invention further provides a pharmaceutical composition which comprises an antibody that immunologically reacts with the present polypeptide.

The invention will now be described in further detail, by way of example only, with reference to the accompanying drawings, in which:

FIG. 1 is a figure of the influence of the present polypeptide on natural killer (NK) activity.

FIG. 2 is a figure of the homology of the present polypeptide in Example 2, IL-lα, IL-lβ, IL-1Rβ and IL-1Raβ.

The present polypeptide is a polypeptide which comprises the amino acid sequence of SEQ ID NO:5 or that has a homology of at least 75% with respect to the amino acid sequence of SEQ ID NO:5 and contains the amino acid sequence of SEQ ID NO:7 as an internal partial amino acid sequence, more particularly, polypeptides comprising or consisting of the amino acid sequence of SEQ ID NO:5 or those comprising or consisting of the amino acid sequence of SEQ ID NO:5 where at least one amino acid is deleted, added and/or replaced with different amino acids at one or more amino acid residues in SEQ ID NO:5, meaning that 1 to 55 amino acid residues in SEQ ID NO:5 are deleted, added and/or replaced with different amino acids can be preferably used as the present polypeptide. These polypeptides according to the present invention have an activity of inhibiting NK activity as their characteristic biological activity.

The present polypeptide can be obtained from human cells or other non-human warm-blooded animal cells, which produce the present polypeptide, and also obtained by genetic engineering techniques. Preferably, the present polypeptide can be easily obtained by culturing to proliferate a human T-cell line capable of producing the present polypeptide, particularly, HPB-MLT cells, FERM BP-2430, in a nutrient culture medium or, as the *in vivo* proliferation method, in the body of non-human warm-blooded animals excluding humans while supplying the animals' body fluids containing nutrients (hereinafter called "body fluid(s)"), and yielding the present polypeptide from the resulting cultures and/or the proliferated cells. The above *in vivo* proliferation method, which employs non-human warm-blooded animals excluding humans, uses new-born rodents such as mice, nude mice, rats, nude rats, guinea pigs, and hamsters. In the method, the warm-blooded animals are injected with anti-thymus antibody derived, for example, from rabbits to suppress their immunoreaction and bred for about 2-10 weeks by conventional methods in general after transplanting to the animals either by subcutaneously or intraperitoneally injecting about 1x10⁵ to 1x10⁸ cells/body of established human cells capable of producing the present polypeptide or by enclosing the human cells in a vessel such as diffusion chambers provided inside or outside the bodies of matured warm-blooded animals and which can circulate the body fluids therethrough. During the breeding, the human cells proliferate while receiving the body fluids. In the case of the present polypeptide is present intra-cellularly, the proliferated cells are collected in the form of a mass of cells, ascites with cells, or cell suspension. If necessary, the cells are successively dispersed or washed with appropriate dispersing solvents and subjected to the following mechanical- or chemical-disruption treatments, followed by collecting the desired present polypeptide from the cell disruptants. The *in vivo* method has the merit that it yields a desired level of proliferated cells at a lesser cost and labor and in shorter period or time than *in vitro* proliferation methods using nutrient culture media. For example, Japanese Patent Kokoku No. 54,158/81 describes in detail the *in vivo* method. To collect from the above cell cultures, the present polypeptide can be yielded from culture supernatants removed from the proliferated cells or prepared from cell disruptants or mixtures of cell disruptants and the culture supernatants, which are obtained by disrupting with or without culture supernatants the proliferated cells, which are separated or unseparated from the cultures, in such a manner of treating with ultrasonic, homogenizing, freeze-thawing, or soaking in hypotonic solvents. The present polypeptide can be collected from the cell disruptants or the mixtures by one or more techniques generally used in this field to separate and purify proteins, for example, salting out, dialysis, filtration, concentration, separatory sedimentation, gel filtration chromatography, ion-exchange chromatography, hydrophobic chromatography, adsorption chromatography, affinity chromatography, chromatofocusing, gel electrophoresis or isoelectric focusing. Depending on purposes, the collected solutions containing the present polypeptide can be concentrated and freeze-dried into a liquid or solid preparation containing the present polypeptide. By appropriately combining two or more of the above techniques, the present polypeptide can be purified to a substantially pure level, i.e., a level which enables to accurately analyze its amino acid sequence.

The present polypeptide can be obtained by applying conventional genetic engineering techniques to the DNAs encoding the present polypeptide. The present polypeptide includes the recombinant DNAs thus obtained as long as they comprise the amino acid sequence of SEQ ID NO:5, or have a homology of at least 75% with respect to the amino acid sequence of SEQ ID NO:5 and include the amino acid sequence of SEQ ID NO:7 as an internal amino acid sequence at the N-terminus. The peptide fragments of the present polypeptide mean those which are obtainable by cleaving the present polypeptide with enzymes or reagents and synthesized by the polypeptide syntheses.

The DNAs encoding the present polypeptide mean those which comprise the nucleotide sequence of SEQ ID NO:4, those which have a homology of at least 75% with respect to the nucleotide sequence of SEQ ID NO:4 and have a nucleotide sequence consisting of the nucleotide sequence of SEQ ID NO:8 at the 5'-terminus, or those which can be obtained by applying conventional chemical syntheses, based on complementary nucleotide sequences to the above nucleotide sequences. In any case, once the DNAs according to the present invention can be obtained, they are easily obtainable by multiplying to a desired level by applying the PCR method or other methods using autonomously-replicable vectors. The DNA fragments, obtainable by fragmenting the above DNAs with conventional methods, are useful for screening the present polypeptide and for preparing antibodies against the present polypeptide by using genetic engineering techniques. The genetic engineering techniques for producing such antibodies *per se* are well known as useful methods for preparing a large amount of humanized antibodies.

The DNAs encoding the present polypeptide include those which are in the form of a recombinant DNA where the present polypeptide has been introduced into autonomously-replicable vectors. Once the desired DNAs are available, the above recombinant DNAs can be easily prepared by conventional genetic engineering techniques. The vectors usable in the present invention include those which are autonomously-replicable in appropriate hosts: For example, pUC18, pBluescript II SK (+), pKK223-3, λgt•λC, etc., which use microorganisms of the genus *Escherichia* as a host; pUB110, pTZ4, pC194, p11, ø1, ø105, etc., which use microorganisms of the genus *Bacillus;* and pHY300PLK, pHV14, TRp7, YEp7, pBS7, etc., which use at least two types of microorganisms as hosts. To incorporate the present polypeptide into the above vectors, conventional methods used in this art can be used in such a manner that the DNAs encoding the present polypeptide obtained in the above are cleaved with restriction enzymes and/or ultrasonics, and then the cleaved DNA fragment encoding the present polypeptide and the cleaved vector fragment are ligated. When used to cleave DNAs, restriction enzymes, which specifically act on nucleotide sequences, such as *Kpn*I, *Acc*I*, Bam*HI, *Bst*XI, *Eco*RI*, Hind*III, *Not*I*, Pst*I, *Sac*I*, Sal*I*, Sma*I, *Spe*I, *Xba*I, *Xho*I, etc., facilitate to ligate the DNA fragment encoding the present polypeptide and a vector fragment. If necessary, these fragments can be ligated after annealing them first and then subjecting the annealed product to the action of a DNA ligase *in vivo* or *in vitro.* The recombinant DNAs thus obtained are infinitely proliferative in appropriate hosts.

The DNAs encoding the present polypeptide include those in the form of a transformant obtainable by introducing the DNAs into appropriate hosts. The transformants can be obtained by introducing the aforesaid DNAs or recombinant DNAs into appropriate hosts to transform them. As the hosts, cells from microorganisms, plants and animals generally used in this field can be selected depending on the vectors used in the recombinant DNAs. Any microorganisms of the genus *Escherichia, Bacillus,* and *Arthrobacter,* as well as *Actinomycoses,* yeasts, and fungi can be arbitrarily used in the present invention. To introduce into such host microorganisms the DNAs according to the present invention, conventional methods such as the competent cell method and the protoplast method can be used. In the transformants according to the present invention, the DNAs encoding the present polypeptide can be present separately from or incorporated into the host's chromosomes. The DNAs incorporated into the host's chromosomes have the merit that they are stably retained in the hosts and may be useful in producing the present polypeptide in a recombinant form. The cultures of the transformants can be arbitrarily treated directly with any of the aforesaid cell disruption methods and then treated with any of the aforesaid separation methods to obtain cell extracts containing the present polypeptide. By using appropriately conventional purification methods for proteins, the present polypeptide with a relatively-high purity can be obtained from the cell extracts. Affinity chromatography using the antibody according to the present invention, which immunologically reacts with the present polypeptide, facilitates the industrial production of the present polypeptide with a relatively-high purity.

The term DNA fragments as referred to in the present invention mean DNA fragments obtainable by cleaving with enzymes or reagents the aforesaid DNAs encoding the present polypeptide, or they can be obtainable by chemical syntheses.

The antibodies which immunologically react with the present polypeptide, particularly, monoclonal antibodies can be prepared by using as antigens the present polypeptide or antigenic fragments thereof, for example, by preparing hybridomas using infinitely-proliferative mammalian cells and antibody-producing cells collected from mammals which had been immunized with such antigens, selecting a clone of hybridoma capable of producing the present monoclonal antibody, and culturing the clone *in vitro* to produce the desired monoclonal antibody. The above antigens are preparable by culturing transformants, into which DNAs encoding either the amino acid sequence of SEQ ID NO:5 or homologous amino acid sequences thereunto have been introduced, and usually can be used after complete or partial purification. To obtain antigenic fragments, the above completely or partially purified antigens are allowed to decompose chemically or enzymatically, or can be synthesized by the peptide synthesis based on the amino acid sequence of SEQ ID NO:5. The antibodies, which immunologically react with the present polypeptide, include receptor proteins for the present polypeptide, which are inherently present in living bodies, in an isolated and purified form from the living bodies.

The immunization used in the present invention can be effected in conventional manner: Mammals are intravenously, intradermally, subcutaneously, or intraperitoneally injected with the above antigens alone or in combination with appropriate adjuvants, and fed for a prescribed period of time. The mammals should not be restricted specifically, and any kind of mammals can be employed in the present invention as long as the desired antibody-producing cells can be obtained thereby independently of their family, species, size or sexes. Usually, the mammals are rodents such as rats, mice and hamsters, and among which most preferable ones are selected in view of the compatibility with the later described infinitely-proliferative mammalian cells. Varying depending on the family, species or size of mammals used, the inoculation amount of the antigen(s) is generally about 5 to 500 µg/head in total at a dose of 2-5 and at an interval of about 1-2 weeks. On 3-5 days after the final inoculation, the spleens are removed from the mammals and dispersed to obtain spleen cells as antibody-producing cells. In this case, sera or anti-sera containing antibodies, which immunologically react with the present polypeptide, can be collected.

By fusing the antibody-producing cells and the infinitely-proliferative mammalian cells thus obtained, hybridization products containing the desired hybridomas can be obtained. Examples of the infinitely-proliferative mammalian cells generally include mouse myeloma cell lines such as P3/NS1/1-Ag4-1 (NS-1) cells, ATCC TIB18; P3X63Ag8 cells, ATCC TIB9; Sp2/0-Ag14 cells, ATCC CRL1581; and mutants thereof. The cell fusion usable in the present invention includes conventional methods using electric pulses and fusion-accelerators such as polyethylene glycol and Sendai virus (HVJ). For example, the cell fusion can be effected by suspending in a cell-fusion medium containing a fusion-accelerator both antibody-producing cells and infinitely-proliferative mammalian cells in a cell ratio of about 1:1 to 1:10, and incubating the cell suspension at a temperature of 30-40°C for about 1-5 min. The cell-fusion media usable in the present invention include media in general such as MEM medium, RPMI1640 medium, and Iscove modified Dulbecco's medium, however, serum should preferably be removed.

To select the desired clone, the cell-fused products obtained in the above are transferred to selection media such as HAT medium and incubated at a temperature of 30-40°C for about three days to three weeks to die cells other than hybridomas. The resulting hybridomas are cultured in a usual manner, and the antibody secreted in the culture is detected by examining the reactivity of the antibody with the present polypeptide. The examination can be effected by conventional method used for detecting antibody such as enzyme immunoassay, radioimmunoassay, and bioassay. For example, *"Tan-Clone-Kotai-Jikken-Manual* (Experimental Manual for Monoclonal Antibody)", edited by Sakuji TOYAMA and Tamie ANDO, published by Kodansha Scientific, Ltd., Tokyo, Japan, pp. 105-152 (1992) describes various methods for detecting antibodies. Hybridomas, which produce antibodies specific to the present polypeptide, can be cloned by the limiting dilution method to isolate a monoclone.

The monoclonal antibody according to the present invention can be obtained by culturing the above hybridomas *in vivo* or *in vitro.* For the culture conventional methods for culturing mammalian cells can be used: For example, in the case of culturing the hybridomas *in vitro,* the monoclonal antibody is collected from the cultures, while in the case of culturing the hybridomas *in vivo* after transplanting into warm-blooded animals excluding humans, the monoclonal antibody is collected from the animals' ascites and/or blood. Conventional methods used to purify antibodies in general can be used to collect the monoclonal antibody from the cultures and animal's ascites or blood. Examples of such are salting out, dialysis, filtration, concentration, centrifugation, separatory sedimentation, gel filtration chromatography, ion-exchange chromatography, affinity chromatography, high-performance liquid chromatography (HPLC), gel electrophoresis, and isoelectric focusing, and if necessary, two or more of these techniques can be used in combination. The resultant purified monoclonal antibody can be concentrated or dried into specimens in the form of a liquid or solid to suit to their use.

The monoclonal antibody according to the present invention is extremely useful for purifying the present polypeptide by immuno affinity chromatography. Such a purification technique comprises the steps of contacting the monoclonal antibody with a mixture containing the present polypeptide and impurities such as proteins other than the present polypeptide to adsorb the present polypeptide on the antibody, and desorbing the present polypeptide therefrom. These steps are generally carried out in an aqueous system. The monoclonal antibody is generally used in an immobilized form fixed to gel water-insoluble carriers which are packed in cylindrical columns. For example, when cultures of transformants or their partially purified products are fed to the columns, only the present polypeptide is substantially adsorbed on the monoclonal antibody and can be desorbed from the antibody by altering the pH around the antibody. For example, in the case of using the monoclonal antibody of the class IgG, the present polypeptide absorbed on the antibody is eluted from the antibody at acid pH conditions, usually, pHs of 2-3. While in the case of using the monoclonal antibody of the class IgM, the present polypeptide adsorbed on the antibody is eluted from the antibody at alkaline pH conditions, usually, pHs of 10-11. The purification method using the monoclonal antibody according to the present invention enables a relatively-high purification of the present polypeptide at a minimum labor and time.

The monoclonal antibody according to the present invention has a relatively-wide applicability to a variety of fields which need the detection of the present polypeptide. When used in labelled immunoassays such as radioimmunoassay, enzyme immunoassay, and fluorescent immunoassay, the monoclonal antibody detects and quantifies the present polypeptide in test samples promptly and accurately. In the assays, the monoclonal antibody according to the present invention is labelled, for example, with radioisotopes, enzymes and/or fluorescent substances prior to use. The monoclonal antibody according to the present invention specifically reacts with the present polypeptide to exhibit an immunoreaction and detects at a relatively-high accuracy a minimum amount of the present polypeptide in test samples with an index of the immunoreaction level for the labelled substances. As compared with bioassay, labelled immunoassay has the following features: It can assay many samples in parallel, reduce the assaying time and labor, and provide data with a relatively-high accuracy. Thus, the detection method according to the present invention is useful for controlling the production steps for the present polypeptide and for the quality control of the final products. Although the present invention does not describe in detail the techniques for labelling monoclonal antibody or labelling assay because this invention does not in itself relate to such an invention, these techniques are described in detail in *"Enzyme Immunoassay",* edited by P. Tijssen, translated by Eiji ISHIKAWA, published by Tokyo-Kagaku-Dojin, pp. 196-347 (1989).

Explaining now the pharmaceutical compositions according to the present invention, since the present polypeptide has inhibitory activity on NK activity which deeply relates to the immune system of mammals, the present polypeptide alone or in combination with one or more conventional NK activity inhibitors, immunomodulators, and physiologically active substances, as well as pharmaceutically-acceptable carriers can be formulated into pharmaceutical compositions as NK activity inhibitors, if necessary. The dose of the present polypeptide to be incorporated into the above pharmaceutical compositions is generally chosen from 0.001-100 µg/adult/day, preferably 0.01-10 µg/adult/day, and more preferably 0.1-1 µg/adult/day. Examples of the form of the pharmaceutical compositions are those in the form of a liquid, powder, tablet, granule, paste, etc., and preferably be formulated into a dose unit form. In addition, the pharmaceutical compositions according to the present invention include those in the form of a composition in general where the antibody according to the present invention immunologically reacts with the present polypeptide.

The DNAs encoding the present polypeptide can be applied for so called gene therapy: In conventional gene therapy, the DNAs according to the present invention can be either inserted, for example, into vectors from viruses such as retrovirus, adenovirus, and adeno-associated virus; or embedded in cationic polymers or liposomes such as a membrane-fused liposome; then directly injecting the resulting DNAs into patients in need of NK activity inhibition or introducing the DNAs into lymphocytes collected from such patients, and transplanting the resulting lymphocytes to the same patients. In adoptive immuno gene therapy, the DNAs according to the present invention can be introduced into effector cells similarly as in conventional gene therapy to treat the aforesaid patients and others suffering from immunodeficiency.

The following Experiments explain the present invention in more detail:

### Experiment 1

### Preparation of mRNA from HPB-MLT cells

A seed culture of a human T-cell, HPB-MLT cells, FERM BP-2430, was inoculated and cultured in RPMI1640 medium, followed by yielding 1.1 g cells by wet weight. The cells were soaked in 7.7 ml of a mixture solution of 5M guanidine isothiocyanate, 10 mM EDTA, 50 mM Tris-HCl buffer (pH 7.0), and 8% (w/v) β-mercaptoethanol, disrupted by a homogenizer, and allowed to stand at 4°C for 15 hours. According to conventional manner, one milliliter of 100 mM EDTA (pH 7.5) containing 5.7 M cesium chloride was placed in a 35-ml centrifugation tube, followed by overlaying over the liquid surface of the tube 10 ml of the above cell disruptant and centrifuging the tube at 25,000 rpm and at 20°C for 20 hours to collect a fraction of RNAs. The fraction was placed in a 15-ml centrifugation tube, shaken for five minutes, centrifuged at 15,000 rpm and at 4°C for 10 min. Thereafter, the formed aqueous layer was collected, admixed with 2.5-fold volumes of ethanol, and allowed to stand at 20°C for two hours to sediment the whole RNAs. The sediments were collected, washed with 75% (v/v) aqueous ethanol solution, and dissolved in 0.5 ml sterilized distilled water to collect the whole RNAs. Using "Oligotex-d(T)₃₀ <SUPER>", an oligo-bound bead commercialized by Japan Synthetic Rubber Co., Ltd., Tokyo, Japan, the whole RNAs were purified to obtain mRNAs from HPB-MLT cells for use in the following experiments.

### Experiment 2

### Preparation of cDNA of polypeptide

### Experiment 2-1

### Preparation of partial cDNA of polypeptide

One microgram of the above mRNAs was placed in a 0.5-ml tube and incubated at 70°C for five minutes by using "DNA Thermal Cycler 480", a thermal cycler commercialized by Perkin-Elmer Corp., Instrument Div., Norwalk, USA, and then instantly cooled to 4°C. To the resultant were added 2 µl 10 x PCR reaction solution, 2 µl of 25 mM magnesium chloride, 2 µl 100 mM dithiothreitol, 1 µl of 2.5 mM dNTPs, 1 µl of 0.2 µg/pl random hexamer, 0.5 µl of 35 U/µl of "RNasin", a ribonuclease inhibitor commercialized by Promega Corporation, Woods Hollow Road, Madison, USA, and 1 µl of 200 U/µ of "Avian Myeloblastosis Virus (AMV) Reverse Transcriptase", a reverse transcriptase commercialized by Japan Synthetic Rubber Co., Ltd., Tokyo, Japan, and the mixture was brought up to a total volume of 20 µl with sterilized distilled water. The resulting mixture was successively incubated at 25°C for 10 min, at 42°C for 30 min, at 99°C for 5 min to react the reverse transcriptase and to obtain an aqueous solution containing a first strand cDNA. cDNA Nucleotide sequences of human IL-1α with accession No. E01146, human IL-1β with accession No. E00619, and human IL-1Ra with accession No. M55646 were obtained from the Gene Bank Data Base and subjected to homology search using a computer software, GENETYX-MAC VER.9.0. For the nucleotide sequence regions which were comparatively preserved in the IL-1α, IL-1β and IL-1Ra, sense primer 1, i.e., s1;5'-(C/G)(T/A)CTACAG(C/T)TG(G/C/A/T)(A/C)(G/C)A-3'; and antisense primer 1, i.e., a1;5'-(G/T)(C/G)GGC(A/C)GAC(T/G)C(A/C)(A/T)(A/G)-3' were constructed and subjected to PCR. Five microliters of 10 x Pfu reaction solution commercialized by Stratagene, CA, USA, 1 µl of 2.5 U/ml Pfu polymerase, 4 µl of 2.5 mM dNTPs (100 ng/µl), 1 µl of s1 primer, and 1 µl of 100 ng/µl of a1 primer were mixed and brought up to a total volume of 50 µl with sterilized distilled water, followed by 30 cycles of a successive incubation at 94°C for 45 sec, at 42°C for 45 sec, and at 72°C for 210 sec. The resulting PCR product was electrophoresed on 2.0% agarose gel to detect a band at a position around 100 bp. The amplified fragments around the position were cut out from the gel and purified on "QIAEX II Gel Extraction Kit", an extraction kit commercialized by Qiagen, K.K., Tokyo, Japan, in such a manner that the desired gel fragments were placed in a 1.5-ml tube, successively admixed with 300 µl of "QX1" solution and "QIAEX II", a DNA adsorption gel, which were enclosed in the above extraction kit, and incubated at 50°C for 10 min. The resulting mixture was centrifuged at 13,000 rpm for 30 sec, and the sediment separated from the supernatant was admixed with and suspended in 500 µl of QX1 solution, and centrifuged at 13,000 rpm for 30 sec for washing the sediment. Thereafter, the resulting sediment was admixed with 500 µl of PE solution and washed twice under the similar centrifugal conditions, followed by removing the supernatant and drying the sediment at ambient temperature for 15 min. Finally, the resulting dried sediment was mixed with 20 µl of 10 mM Tris-HCl buffer containing 1 mM EDTA, i.e., TE buffer (pH 8.0), allowed to stand at ambient temperature for 5 min to extract DNAs, and centrifuged at 13,000 rpm for 30 sec to obtain the desired DNA fragments.

Using "pCR-Script SK(+) Cloning Kit", a cloning kit commercialized by Stratagene, CA, USA, DNA fragment-1 was cloned. In a 0.5 ml tube were placed one microliter of 10 ng/µl of pCR-Script SK(+) cloning vector, 1 µl of pCR-Script 10 x reaction solution, 0.5 µl of 10 mM rATP, 4 µl of 5 U/µl of the DNA fragment-1, 1 µl of 5 U/µl of SrfI restriction enzyme, and 1 µl of 4 U/µl of T4DNA ligase, and the mixture was brought up to a total volume of 10 µl with sterilized distilled water. The resulting mixture was allowed to react for one hour at ambient temperature to effect ligation, followed by incubating the mixture at 65°C for 10 min to suspend the reaction, then cooling the mixture to 4°C. Thereafter, transformation was carried out by the following method according to conventional manner: Forty microliters of a seed culture of "Epicurian Coli XL1-Blue MRF' Kan supercompetent cells", a strain of *Escherichia,* were placed in a previously-cooled Falcon 2059 polypropylene tube, commercialized by Falcon Labware, Lincoln Park, N.J., USA, 0.7 µl of 1.44 M β-mercaptoethanol, and cooled with ice for 10 min. To the mixture was added 2 µl of the above ligated product, gently stirred, and allowed to stand for 30 min under cooling conditions with ice. After heating at 42°C for 45 sec, the resulting mixture was mixed with 450 µl of SOC medium and incubated under shaking conditions at 37°C for one hour. Thereafter, the culture was inoculated to LB medium containing 1 % sodium chloride, 1 % tryptone, 0.5 % yeast extract, and 2 % agar, supplemented with 0.4 mg of X-gal, i.e., 5-bromo-4-chloro-3-indolyl-β-D-galactopyranoside, and 40 µl of 100 mM isopropyl thiogalactoside, and incubated at 37°C for 16 hours. Upon the colony PCR using sl and al primers, a colony detected with the desired 100 bp band was inoculated to LB medium and cultured at 37°C for 16 hours under shaking conditions. With two milliliters of the culture, plasmids were prepared as follows according to conventional manner: The cells were collected from the culture by centrifugation at 3,000 rpm for 5 min, admixed with 100 µl of 10 ng/µl of lysozyme, incubated at ambient temperature for 5 min, admixed with 200 µl of 0.2 M sodium dodecylsulfate, cooled with ice for 5 min, mixed with 150 µl of 5 M potassium acetate, and cooled with ice for 5 min. After centrifugation at 15,000 rpm for 5 min, 400 µl of the supernatant were placed in a 1.5-ml tube, admixed with an equal volume of phenol/chloroform/isoamyl alcohol (=25:24:1 by volume), vigorously shaking for 5 min, and centrifuged at 15,000 rpm for 5 min to remove proteins, followed by collecting 280 µl of the resulting aqueous layer and subjecting to conventional ethanol sedimentation in a usual manner. Thereafter, 60 µl of 40 mg/ml RNase in TE buffer were added to the resulting sediment, incubated at 37°C for 1.5 hours, admixed with 30 µl of 20 % polyethylene glycol, and allowed to stand for 1.5 hours while cooling with ice. The mixture was centrifuged at 15,000 rpm for 30 min, and the sediment separated from the supernatant was washed with 200 µl of cooled 75% (v/v) aqueous ethanol solution, dried for 10 min under reduced pressure, and dissolved in sterilized water. The plasmid thus obtained was analyzed for nucleotide sequence by the deoxy method using "DNA SEQUENCER 373A", a DNA sequencer, commercialized by Applied Biosystems, Inc., Foster City, CA, USA, resulting in provisional determination of the partial nucleotide sequence of SEQ ID NO:1 of the present polypeptide. To clone the cDNA of the present polypeptide as a homo protein, cDNA nucleotide sequences at the 5'- and 3'-termini were determined on "5'/3' RACE Kit", a RACE kit, commercialized by Roche Diagnostics, K.K., Tokyo, Japan.

### Experiment 2-2

### 5' RACE method

5'-ATGTTCCAGGAGCCCACC-3', as antisense primer (a2), positioning at residues 103-86 in SEQ ID NO:1; 5'-AGCCCTATAAAAGATGAAG-3', as antisense primer 3 (a3), positioning at residues 83-65 in SEQ ID NO:1; and 5'-CGGCGTGCTGATTCCTTTTG-3', as antisense primer 4 (a4), positioning at residues 61-42 in SEQ ID NO:1 were constructed. Four microliters of 5 x cDNA synthetic solution containing 250 mM Tris-HCl (pH 8.5), 40 mM magnesium chloride, 150 mM potassium chloride, and 5 mM dithiothreitol; 2 µl of 2.5 mM dNTPs, 1 µl of 100 ng/µl of a2 primer, 240 ng of mRNAs from human HPB-MLT cells; and 1 µl of 20 U/µl of a reverse transcriptase from AMV (Avian Myeloblastosis Virus), and the mixture was brought up to a total volume of 20 µl with DEPC-treated water. The resulting mixture was incubated at 55°C for one hour and then at 65°C for 10 min to obtain a cDNA specimen. The cDNA specimen was purified using a spin column in such a manner that the specimen was placed in the spin column along with 100 µl of a reaction solution consisting of 3 M guanidine thiocyanate, 10 mM Tris-HCl (pH 6.6), and 5% ethanol, and the mixture was centrifuged at 15,000 rpm for 30 sec. To the spin column was added 500 µl of a washing solution consisting of 20 mM sodium chloride and 2 mM Tris-HCl (pH 7.5) in ethanol, and the mixture was washed by centrifugation at 15,000 rpm for 30 sec and similarly treated after the addition of 200 µl of a fresh preparation of the same washing solution. The sediment thus obtained was admixed with 50 µl of an extracting solution containing 10 mM Tris-HCl (pH 8.5) and 1 mM EDTA and centrifuged at 15,000 rpm for 30 sec to obtain a purified cDNA specimen. To 19 µl of the purified cDNA specimen were added 2.5 µl of 10 x reaction solution containing 100 mM Tris-HCl (pH 8.3), 15 mM magnesium chloride, and 500 mM potassium chloride, and 2.5 µl of 2 mM dATP to give a total volume of 24 µl. The mixture thus obtained was heated at 94°C for 3 min, then cooled promptly, admixed with 1 µl of 10 U/µl of a terminal transferase, and successively incubated at 37°C for 30 min and at 72°C for 10 min. Five microliters of the resulting synthesized product, 1 µl of 37.5 µM of oligo-dT-anchor-primer (5'-GACCACGCGTATCGATGTCGACTTTTTTTTTTTTTTTTV-3', where V is A, C or G), 1 µl of 100 ng/µl of a3 primer, 1 µl of 25 mM dNTPs, 1 µl of 2.5 U/ml of Pfu polymerase, and 5 µl of 10 x Pfu reaction solution were mixed, and the mixture was brought up to a volume of 50 µl with sterilized distilled water and treated with 35 cycles of successive incubations at 94°C for 45 sec, 55°C for 45 sec, and 72°C for 2 min to amplify the synthesized product. One microliter of the amplified product was provided and amplified under the similar conditions as above, where 12.5 M PCR anchor primer (5'-GACCACGCGTATCGATGTCGAC-3') and 90 ng/µl of a4 primer were used in this re-amplification. Electrophoresis of the resulting PCR product on 1.2 % agarose gel detected a band at a position of about 550 bp. Thereafter, the detected fragment in the band was similarly purified as above on "QIAEX II Gel Extraction Kit", an extraction kit commercialized by Qiagen, K.K., Tokyo, Japan, and cloned using "pCR-Script SK(+) Cloning Kit", a cloning kit commercialized by Stratagene, CA, USA. According to conventional manner, "Epicurian Coli XL1-Blue MRF' Kan supercompetent cells", a strain of *Escherichia* commercialized by Stratagene, CA, USA, was transformed with the above cloned product. The transformants thus obtained were cultured in a nutrient culture medium to form colonies, and the desired colony with a prescribed band detected by the colony PCR was selected, inoculated into LB-liquid medium, and cultured at 37°C for 16 hours under shaking conditions. From 2 ml of the culture, a plasmid, named "pCRILL-5", was prepared and analyzed for nucleotide sequence by the dideoxy method using a DNA sequencer. As a result, a nucleotide sequence of about 575 bp of SEQ ID NO:2 in the 5'-terminal region of cDNA was obtained.

### Experiment 2-3

### 3' RACE Method

As sense primer 2 (s2), 5'-CTGATGAAGCTGGCTGCC-3' positioning at residues 24-42 in SEQ ID NO:1, was constructed. Four microliters of 5 x cDNA synthetic solution, 2 µl of 2.5 mM dNTPs, 1 µl of 37.5 µM oligo-dT-anchor-primer, 540 ng of mRNAs from human HPB-MLT cells, and 1 µl of 20 U/µl of a reverse transcriptase from AMV were mixed, and the mixture was brought up to a total volume of 20 µl with DEPC-treated water, and reacted by using thermal cycler at 55°C for 60 min and at 65°C for 10 min to obtain a DNA product. One microliter of the DNA product was mixed with 1 µl of 12.5 µl of PCR anchor-primer (5'-GACCACGCGTATCGATGTCGAC-3'), 1 µl of 2.5 U/µl of Pfu polymerase, and 5 µl of 10 x Pfu reaction solution were mixed, and the mixture was brought up to a total volume of 50 µl with sterilized distilled water. The mixture was treated with 35 cycles of successive incubations at 94°C for 45 sec, 55°C for 45 sec, and 72°C for 120 sec to amplify the DNA product. Electrophoresis of the amplified product on 1.2 % agarose gel detected a band at a position of about 320 bp. Similarly as above, the band with a fragment of the PCR product was purified on "QIAEX II Gel Extraction Kit", an extraction kit commercialized by Qiagen K.K., Tokyo, Japan, and cloned by using "pCR-Script SK(+) Cloning Kit", a cloning kit commercialized by Stratagene, CA, USA. According to conventional manner, "Epicurian Coli XLl-Blue MRF' Kan supercompetent cells", a strain of *Escherichia* commercialized by Stratagene, CA, USA, was transformed with the cloned product. After culturing in a nutrient culture medium, a colony with the desired band was selected by using the colony PCR, then inoculated into LB-liquid medium and cultured at 37°C for 16 hours under shaking conditions to form colonies. From the colonies, a plasmid, named "pCRILL-3", was isolated in a usual manner, and analyzed for nucleotide sequence by the dideoxy method, resulting in a nucleotide sequence of 248 bp of SEQ ID NO:3 as a nucleotide sequence of the 3'-terminal region of cDNA of the present polypeptide.

### Experiment 2-4

### Preparation of cDNA of polypeptide

Based on the nucleotide sequences obtained in Experiments 2-2 and 2-3, the 5'-terminal sense primer s4 (5'-GAGAACTGAAGGCAAACAG-3') and the 3'-terminal antisense primer a5 (5'-AGTGAGCAGGTTTGGGGTT-3') of the cDNA of the present polypeptide were constructed and amplified by the RT-PCR method to obtain the homo nucleotide sequence of the present polypeptide: Four microliters of 5 x cDNA synthetic solution, 2 µl of 2.5 mM dNTPs, 1 µl of 37.5 µM oligo-dT-anchor-primer, 540 ng of mRNAs from HPB-MLT cells, and 1 µl of 20 U/µl of a reverse transcriptase from AMV were mixed, and the mixture was brought up to a volume of 20 µl with DEPC treatment solution and reacted by incubating at 55°C for 60 min and 65°C for 10 min by using thermal cycler. To 1 µl of the resulting cDNA were added 1 µl of 100 ng/µl aliquots of sense primer s4 and antisense primer a5, 1 µl of 25 mM dNTPs, 1 µl of 2.5 U/µl of Pfu polymerase, and 5 µl of 10 x Pfu reaction solution, and the mixture was brought up to a volume of 50 µl with sterilized distilled water and treated with 35 cycles of successive incubations at 94°C for 45 sec, 55°C for 45 sec, and 72°C for 120 sec to amplify the cDNA. Electrophoresis of the amplified product on 1.2 % agarose gel detected a band at a position of about 850 bp. Similarly as above, the band with a fragment of the amplified product was purified on "QIAEX II Gel Extraction Kit", an extraction kit commercialized by Qiagen K.K., Tokyo, Japan, and cloned by using "pCR-Script SK(+) Cloning Kit", a cloning kit commercialized by Stratagene, CA, USA. According to conventional manner, "Epicurian Coli XL1-Blue MRF' Kan supercompetent cells", a strain of *Escherichia* commercialized by Stratagene, CA, USA, was transformed with the cloned product. The transformants thus obtained were cultured in a nutrient culture medium to form colonies, and the desired colony with a prescribed band detected by the colony PCR was selected, inoculated into LB-liquid medium, and cultured at 37°C for 16 hours under shaking conditions. From 2 ml of the culture, a plasmid was obtained in a usual manner and named "pCRILL-full". The nucleotide sequence of the plasmid was analyzed by the dideoxy method using a DNA sequencer to determine the cDNA of the present polypeptide as shown in SEQ ID NO:4. The amino acid sequence of the present polypeptide for the nucleotide sequence of SEQ ID NO:4 is as shown in SEQ ID NO:5.

### Experiment 3

### Biological activity

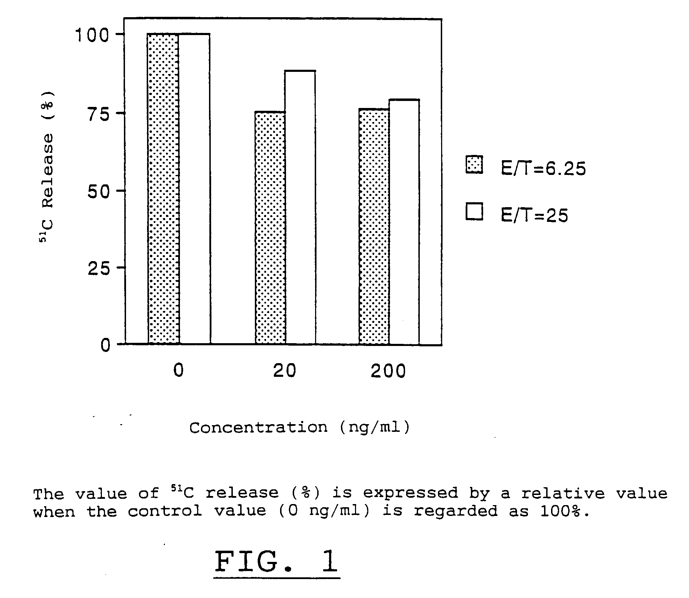

According to conventional manner, peripheral blood was collected from a healthy donor and treated with "FICOL", a watersoluble non-ionic polymer commercialized by Pharmacia LKB Biotechnology AB, Uppsala, Sweden, to obtain peripheral lymphocytes containing NK cells, followed by washing twice with RPMI1640 medium supplemented with 0.1 mM β-mercaptoethanol and 10% (v/v) fetal calf serum and suspending into a 5 x 10⁶ cells/ml of cell suspension. The cell suspension was distributed to 96-well microliter plates in a volume of 100 µl/well, and to each well was added both 50 µl of a solution with a prescribed concentration of a specimen of the present polypeptide obtained in the following Example 2 and 50 µl of a fresh preparation of the same medium as above, followed by the incubation at 37°C for 18 hours. After the incubation, peripheral lymphocytes containing NK cells were collected and mixed with ⁵¹Cr-labelled K-562 cells, ATCC CCL243, in a ratio of 6.25 and 25 (=(Peripheral lymphocytes containing NK cells as effector cell (E))/(K-562 cells as target cells (T)), a ratio of E/T), and incubated at 37°C for 4 hours. After the incubation, the 96-cell microliter plates were centrifuged at 2,000 rpm for 5 min, followed by counting ⁵¹Cr in each supernatant of the wells to examine the influence of the present polypeptide on the NK activity. The results are in FIG. 1.

As evident from the results in FIG. 1, as compared with the peripheral lymphocytes containing NK cells without addition of the present polypeptide, those with the present polypeptide showed a significant reduction of the count of ⁵¹Cr in each culture supernatant, indicating that the present polypeptide inhibits the NK cell activity.

### Example 1

### Preparation of recombinant DNA and transformant

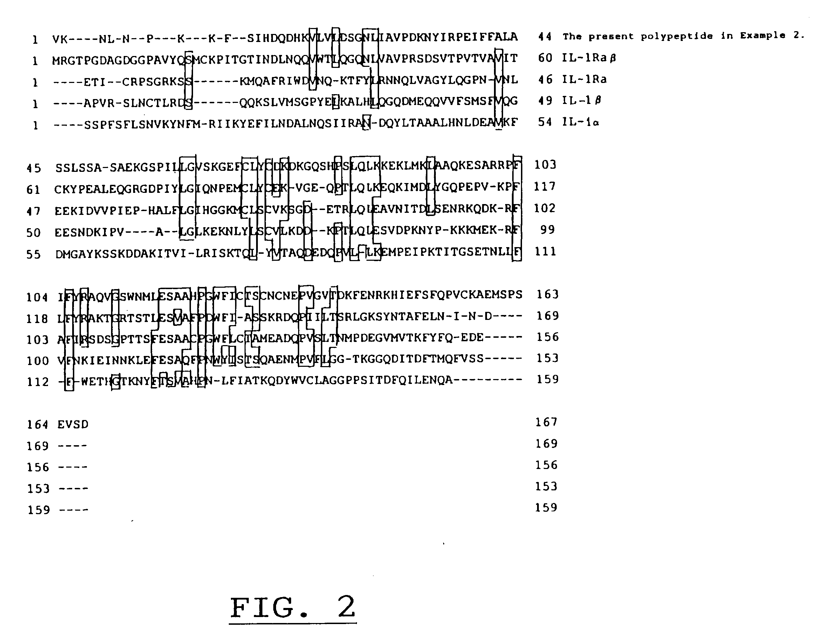

The following recombinant DNA was prepared to express in a system with a microorganism of the genus *Escherichia* a polypeptide, as the present polypeptide, consisting of 167 amino acid residues corresponding to the amino acid residues from valine residue 52 of the amino acid sequence, encoded by the cDNA of the present polypeptide as shown in SEQ OD NO:5, to aspartic acid residue at the C-terminal amino acid residue of the present polypeptide: To a 0.5-ml reaction tube were added 10 µl of 10 x Pfu reaction solution commercialized by Stratagene, CA, USA, 1 µl of 2.5 U/ml of Pfu polymerase, 1 µl of 25 mM dNTPs, 1 ng of the above plasmid pCRILL-full, and adequate amounts of a sense primer of a nucleotide sequence of 5'-ACTGCATATGGTGAAGAACTTAAACCCG-3', consisting of random 4 bases with the following *Nde*I site and a nucleotide sequence corresponding to residues 274-291 of SEQ ID NO:4; and an antisense primer of a nucleotide sequence of 5'-ATGCGGATCCTTACTAATCGCTGACCTC-3', consisting of random 4 bases with the following *Bam*HI site, a terminal codon, and a nucleotide sequence corresponding to residues 777-763 of SEQ ID NO:4. The mixture was brought up to a volume of 50 µl with sterilized distilled water. Thereafter, the resulting mixture was subjected to PCR reaction by treating with 30 cycles of successive incubations at 94°C for 0.5 min, 60°C for 1 min, and 72°C for 1 min. The resulting PCR product was in a usual manner digested with restriction enzymes *Nde*I and *Bam*HI at 37°C for 2 hours and treated by heating at 70°C for 15 min to inactivate the enzymes. While "pET-3a", a plasmid commercialized by Novagen, Madison, USA, was similarly digested with restriction enzymes *Nde*I and *Bam*HI at 37°C for 2 hours and treated by heating at 70°C for 15 min. One hundred nanograms of "pET-3a" as a vector treated similarly as above with the restriction enzymes and 200 ng of the PCR product treated similarly as above with the restriction enzymes were ligated using "DNA Ligation Kit Ver. 2", a ligation kit commercialized by Takara Shuzo Co., Ltd., to construct an expression plasmid, pETILL-EX. According to conventional manner, "BL21 (DE3) pLysS", a microorganism of the genus *Escherichia* commercialized by Novagen, Madison, USA, was transformed with pETILL-EX to obtain a transformant named "ILL-EX-1". The amino acid sequence used in this experiment, which consists of 167 amino acid residues corresponding to the amino acid residues from valine residue 52 of the amino acid sequence encoded by the cDNA of the present polypeptide as shown in SEQ ID NO:5, has only a homology of about 20% to those of IL-1α, IL-1β, IL-lRa, and IL-1Raβ as shown in FIG. 2. In FIG. 2, each alphabet is a single character code of amino acid.

### Example 2

### Preparation of polypeptide

The transformant ILL-EX-1 in Example 1 was inoculated into 5 ml LB medium (pH 7.2) containing 50 µg/ml ampicillin and 20 µg/ml chloramphenicol, and incubated at 37°C for 18 hours. After the culture, 1 ml of the culture was collected, inoculated into 100 ml LB medium containing the above two antibiotics, cultured at 37°C for 4 hours, admixed with 1 ml of 100 mM IPTG, isopropyl-β-D-galactopyranoside, and further incubated for 2 hours, followed by centrifugation at 10,000 rpm for 15 min to collect the proliferated cells. The pasty cells were admixed with 10 ml of 10 mM phosphate buffer (pH 6.8) containing 0.5 M urea and 0.1% β-mercaptoethanol and stirred at ambient temperature for 3 hours. After stirring, the cell suspension was centrifuged at 10,000 rpm for 20 min to collect the cells. To the cells was added 10 ml of 10 mM sodium phosphate buffer (pH 6.8) containing 8 M urea and 0.1% β-mercaptoethanol, followed by stirring at ambient temperature for 18 hours and centrifuging the resulting mixture at 10,000 rpm for 20 min to collect a supernatant. The supernatant thus obtained was dialyzed at 4°C for 18 hours against 1 ℓ of 10 mM sodium phosphate buffer (pH 6.8) containing 0.5 M urea and 0.1 % β-mercaptoethanol, and centrifuged at 10,000 rpm for 20 min to collect a supernatant. The supernatant was then subjected to gel filtration using "SUPERDEX 75", a gel for chromatography commercialized by Pharmacia LKB Biotechnology AB, Uppsala, Sweden, and using PBS with 0.1% β-mercaptoethanol as an eluent to collect a fraction corresponding to a molecular weight of about 20 kDa. Thus, a purified specimen of the present polypeptide was obtained.

### Example 3

### Preparation of hybridoma and monoclonal antibody

### Example 3-1

### Preparation of hybridoma

BALB/c mice, 10-week-old, were intraperitoneally injected by a syringe with 20 µg/mouse of a purified specimen of the present polypeptide, obtained by the method in Example 2, along with complete Freund's adjuvant. The mice were received twice with such an injection at an interval of two weeks over four weeks, and further received with additional injection one week after the final injection. On three days after the final shot, the spleens were removed from the mice and dispersed in physiological saline into a suspension of spleen cells.

The spleen cells and SP2/0-Ag14 cells from mouse myeloma, ATCC CRL1581, were suspended in serum-free RPMI1640 medium (pH 7.2) pre-warmed at 37°C in respective cell concentrations of 3 x 10⁴ cells/ml and 1 x 10⁴ cells/ml, then centrifuged to collect sediments. To the sediments was added drop by drop over one minute 1 ml of serum-free RPMI1640 medium (pH 7.2) containing 50% (w/v) polyethylene glycol with a molecular weight of 1,500 daltons, and the mixture was incubated at 37°C for 1 min, brought up to a total volume of 50 ml by the addition of serum-free RPMI1640 medium (pH 7.2) drop by drop, and centrifuged to collect sediments. The sediments thus obtained were suspended in HAT medium, and the suspension was distributed to 96-well microliter plates in a volume of 200 µl/well and incubated at 37°C for one week to select hybridomas.

For antibody secreted in a supernatant in each well, the reactivity with the purified specimen of the present polypeptide obtained by the method in Example 2 was examined by the enzyme immunoassay, and then hybridomas capable of producing an antibody reactive with the present polypeptide were selected, followed by repeating the limiting dilution in a usual manner to obtain a clone of hybridoma capable of producing the monoclonal antibody according to the present invention.

### Example 3-2

### Preparation of monoclonal antibody

The clone of hybridoma in Example 3-1, capable of producing the present monoclonal antibody, was cultured in a nutrient culture medium, and the culture was purified to yield about 5 mg of a monoclonal antibody, which immunologically reacts with the present polypeptide, per BALB/c mouse.

### Example 4

### Detection of polypeptide by enzyme immunoassay

According to conventional manner, rabbits were immunized with a purified specimen of the present polypeptide obtained by the method in Example 2 and collected blood, followed by purifying and isolating an antibody which immunologically reacted with the purified specimen. The antibody was dissolved in PBS to give a concentration of 20 µg/ml, and the solution was distributed to 96-well microliter plates in a volume of 100 µl/well. The microliter plates were incubated at ambient temperature for three hours, and after removal of the solution containing antibody from each well, poured with PBS containing 1% (w/v) calf serum albumin in a volume of 200 µl/well, and allowed to stand at 4°C overnight.

PBS was removed from the microliter plates, and the wells of the microliter plates were washed with PBS containing 0.05% (v/v) tween 20 and poured with 100 µl/well of solutions of the present polypeptide diluted appropriately with PBS containing 0.5% (w/v) calf serum albumin, followed by immunological reaction at ambient temperature for two hours under shaking conditions. The wells of the microliter plates were washed with PBS containing 0.05% (v/v) tween 20 and poured with 100 µl/well of an antibody which immunologically reacts with the purified specimen of the present polypeptide labelled previously with biotin, followed by immunological reaction at ambient temperature for two hours under shaking conditions. Thereafter, the wells were washed with PBS containing 0.05% (v/v) tween 20, poured with 100 µl/well of a complex of horseradish peroxidase and streptoavidin, and further incubated at ambient temperature for two hours under shaking conditions. Thereafter, the wells were washed with PBS containing 0.05% (v/v) tween 20, followed by measuring the absorbance at a wavelength of 492 nm for the activity of horseradish peroxidase bound to the purified specimen of the present polypeptide using o-phenylenediamine as a substrate for the enzyme. The detection method well detects the present polypeptide at a relatively-high sensitivity and accuracy.

As described above, the present invention provides a polypeptide comprising either the amino acid sequence of SEQ ID NO:5 or an amino acid sequence which has a homology of at least 75% with respect to the amino acid sequence of SEQ ID NO:5 and contains the amino acid sequence of SEQ ID NO:7 as an internal partial amino acid sequence; a DNA comprising a nucleotide sequence of SEQ ID NO:4, a DNA which has a homology of at least 75% with respect to the nucleotide sequence of SEQ ID NO:4 and the nucleotide sequence of SEQ ID NO:8 at the 5'-terminus, and a DNA comprising a complementary nucleotide sequence thereunto; an antibody which immunologically reacts with the present polypeptide; and a method for detecting the present polypeptide. The present invention will contribute to the researches on mammalian immune systems and the development of pharmaceuticals as inhibitors/regulators of NK activity.

Thus, the present invention having such an outstanding functions and effects is a significant invention that contributes to this art.

While there has been described what is at present considered to be the preferred embodiments of the invention, it will be understood the various modifications may be made therein, and it is intended to cover in the appended claims all such modifications as fall within the true spirits and scope of the invention.

## Claims

1. A polypeptide comprising either the amino acid sequence of SEQ ID NO:5 or an amino acid sequence which has a homology of at least 75% with respect to the amino acid sequence of SEQ ID NO:5 and contains the amino acid sequence of SEQ ID NO:7 as an internal partial amino acid sequence:

SEQ ID NO:5

1               5                   10                  15

            20                  25                  30

        35                  40                  45

    50                  55                  60

65                  70                  75                  80

                85                  90                  95

            100                 105                 110

        115                 120                 125

    130                 135                 140

145                 150                 155                 160

                165                 170                 175

            180                 185                 190

        195                 200                 205

    210                 215

SEQ ID NO:7
Val Lys Asn
1

2. A polypeptide fragment obtainable from the polypeptide of claim 1.

3. A receptor protein which binds to the polypeptide of claim 1.

4. A DNA comprising (i) a nucleotide sequence which has the nucleotide sequence of SEQ ID NO:4 encoding the polypeptide of claim 1, (ii) a nucleotide sequence which has a homology of at least 75% with respect to the nucleotide sequence of SEQ ID NO:4 and contains the nucleotide sequence of SEQ ID NO:8 at the 5'-terminus, or (iii) a complementary nucleotide sequence thereunto:

SEQ ID NO:4
gagaactgaa ggcaaacaga gctccaggag tccaagacag agccacagac cacgaggatc 60
cctggcccag gtcttggact tcattccatt ttctgttgag taataaactc aacgttgaaa 120
atgtcctttg tgggggagaa ctcaggagtg aaaatgggct ctgaggactg ggaaaaagat 180
gaaccccagt gctgcttaga agacccggct gtaagccccc tggaaccagg cccaagcctc 240
cccgccatga attttgttca cacaagtcca aaggtgaaga acttaaaccc gaagaaattc 300
agcattcatg accaggatca caaagtactg gtcctggact ctgggaatct catagcagtt 360
ccagataaaa actacatacg cccagagatc ttctttgcat tagcctcatc cttgagctca 420
gcctctgcgg agaaaggaag tccgattctc ctgggggtct ctaaagggga gttttgtctc 480
tactgtgaca aggataaagg acaaagtcat ccatcccttc agctgaagaa ggagaaactg 540
atgaagctgg ctgcccaaaa ggaatcagca cgccggccct tcatctttta tagggctcag 600
gtgggctcct ggaacatgct ggagtcggcg gctcaccccg gatggttcat ctgcacctcc 660
tgcaattgta atgagcctgt tggggtgaca gataaatttg agaacaggaa acacattgaa 720
ttttcatttc aaccagtttg caaagctgaa atgagcccca gtgaggtcag cgattaggaa 780
actgccccat tgaacgcctt cctcgctaat ttgaactaat tgtataaaaa ccccaaacct 840
gctcact 847

SEQ ID NO:8
gtg aag aac 9

5. A recombinant DNA comprising the DNA of claim 4 and an autonomously-replicable vector.

6. A DNA fragment obtainable from the DNA of claim 4.

7. A transformant prepared by introducing the recombinant DNA of claim 5 into an appropriate host.

8. The transformant of claim 7, wherein said host is a microorganisms of the genus *Escherichia.*

9. A process for producing the polypeptide of claim 1, which comprises the steps of proliferating an established T-cell line, which is derived from a human or a warm-blooded animal excluding humans and capable of producing the polypeptide, either in a nutrient culture medium or in the body of a warm-blooded animal excluding humans while supplying the animal's body fluid containing nutrients of said warm-blooded animal, to produce the polypeptide; and collecting the produced polypeptide.

10. The process of claim 9, wherein said established T-cell line is HPB-MLT cells, FERM BP-2430.

11. A process for producing the polypeptide of claim 1, comprising the steps of culturing the transformant of claim 7 or 8 in a nutrient culture medium, and collecting the produced polypeptide from the culture.

12. An antibody which immunologically reacts with the polypeptide of claim 1.

13. The antibody of claim 12, which is a monoclonal antibody.

14. The antibody of claim 12, which is an anti-serum.

15. The antibody of claim 12, which is a humanized antibody.

16. A pharmaceutical composition comprising the polypeptide of claim 1 as an effective ingredient.

17. A pharmaceutical composition comprising the antibody of any one of claims 12 to 15.
